Europäisches Patentamt

European Patent Office

Office européen des brevets

(11) Numéro de publication: **0 237 394**
**A1**

## (12) DEMANDE DE BREVET EUROPEEN

(21) Numéro de dépôt: 87400374.2

(22) Date de dépôt: 20.02.87

(51) Int. Cl.⁴: **C 12 Q 1/34**

(30) Priorité: 21.02.86 FR 8602470

(43) Date de publication de la demande:
16.09.87 Bulletin 87/38

(84) Etats contractants désignés:
AT BE CH DE ES FR GB GR IT LI LU NL SE

(71) Demandeur: ARLEY-GUILLAUBEY LABORATOIRES
25, rue Saint-Ferdinand
F-75017 Paris (FR)

(72) Inventeur: Saunier, Brigitte
10, rue Ernest Psichari
F-75007 Paris (FR)

Potherat, Jean-Jaques
10, place de l'Albinque
F-81100 Castres (FR)

Alessi, Mireille
La Fosse
F-81100 Castres (FR)

Guillaubey, Arlette
36, rue de la Ferme
F-92200 Neuilly-sur-Seine (FR)

Eyquem, André
24, avenue Félix Faure
F-75015 Paris (FR)

(74) Mandataire: Ahner, Francis
CABINET REGIMBEAU 26, avenue Kléber
F-75116 Paris (FR)

(54) Agent de diagnostic et son procédé de préparation pour déceler les infections urogénitales.

(57) Agent de diagnostic permettant de déterminer la présence de leucocytes dans les urines, que ces leucocytes soient consécutifs à une infection urinaire ou à une infection génitale. Cet agent de diagnostic comporte un tube à essai transparent renfermant au moins une bille et, sous forme solide, au moins un ester de N-méthylindoxyle obtenu à partir d'acide carboxylique en $C_2$ à $C_5$.

Procédé de préparation dudit agent de diagnostic, et son utilisation à titre d'agent de détermination de la présence de leucocytes dans les urines.

EP 0 237 394 A1

**Description**

## AGENT DE DIAGNOSTIC ET SON PROCEDE DE PREPARATION POUR DECELER LES INFECTIONS UROGENITALES

La présente invention a pour objet un agent de diagnostic permettant de déterminer la présence de leucocytes dans les urines, que ces leucocytes soient consécutifs à une infection urinaire ou à une infection génitale. Elle a également pour objet un procédé de préparation dudit agent de diagnostic, et son utilisation à titre d'agent de détermination de la présence de leucocytes dans les urines.

D'après GB-A- 1 128 371 on connaît un agent de détermination de la présence d'enzymes hydrolytiques dans des liquides physiologiques et un procédé pour sa préparation consistant à imprégner un support sponieux par des esters d'indoxyle ou de thio-indoxyle incolores, notamment d'un acétate, et éventuellement d'un tampon ou d'un oxydant. En présence d'enzymes hydrolytiques, les esters forment des groupes indoxyle ou thio-indoxyle libres et sous l'action de l'oxygène de l'air, ceux-ci conduisent à la formation d'une coloration foncée. Les composés revendiqués dans ce brevet antérieur ne permettent pas un dosage fiable des leucocytes et ne donnent lieu à aucune réaction même en présence de 10 000 leucocytes/µl (cf. EP-A-012 957).

Par ailleurs, le brevet EP-A-012 957 divulgue des agents de diagnostic, permettant de mettre en évidence la présence de germes protéolytiques dans des liquides physiologiques. Ces agents de diagnostic contiennent un support absorbant, une couche de film, un mélange pulvérulent, un lyophilisat, une substance tampon et une solution ou un comprimé renfermant les réactifs. Ces réactifs consistent en des esters d'indoxyle et/ou de thio-indoxyles avec des amino-acides et/ou des peptides dont l'atome d'azote est protégé par un groupe protecteur. A titre de support absorbant, est employé de préférence du papier-filtre, de la cellulose ou des toisons de fibres synthétiques, que l'on imprègne en plusieurs étapes de solutions comportant les réactifs nécessaires. Ainsi, les diverses étapes d'imprégnation et le grand nombre de constituants utilisés dans de tels réactifs rendent leur procédé de préparation très complexe. En outre, Ces réactifs permettent de mettre en évidence des protéases présentes dans les granulocytes neutrophiles, ainsi que des enzymes protéolytiques comme par exemple l'élastase, la chymotrypsine, la trypsine dans des solutions aqueuses ou des liquides physiologiques tel que plasma, sérum, liquide céphalorachidien, etc. Ces germes protéolytiques sont très différents des enzymes leucocytaires dont la présente invention vise à déceler la présence.

On connaît en outre l'article de **G.G. GUILBAULT** publié par la revue Analytical Letters 1(6), 365-379 (1968) ayant pour titre "N-méthylindoxylesters as substrates for cholinesterase". Cet article se réfère à un article **d'ETTINGER et FRIEDLANDER** dans Ber. 45, 2074 (1912) et à un article de **HOLT** dans Nature 169, 271 (1952) selon lequel l'acétate de N-méthylindoxyle constituerait un substrat fluorogène idéal. Dans l'article de **GUILBAULT** précité, est décrit l'emploi d'acétate, butyrate ou propionate de N-méthylindoxyle pour déterminer la présence de cholinestérase ou de pseudo-cholinestérase (cholinestérase de sérum de cheval ou érythrocytes bovins), cette détermination étant effectuée par mesure de la fluorescence, obtenue après hydrolyse par la cholinestérase du N-méthylindoxyle, dans un spectrofluoromètre. En fait, la méthode de détermination décrite dans cet article de **GUILBAULT** nécessite l'emploi d'un appareillage relativement compliqué. En outre, les esters de N-méthylindoxyle en solution sont fort instables. Au surplus, cet article de **GUILBAULT** ne mentionne en aucune façon l'emploi d'esters de N-méthylindoxyle pour la détermination de la présence de leucocytes dans des urines.

On connaît enfin un article de **E.VORMITTAG** et al dans Chemical Abstracts vol.96, no 5, 1er février 1982, page 239, no 30471d, Columbus, Ohio, US, relatif à la détection des leucocytes dans l'urine au moyen d'une bande réactive ; il s'agit d'une réaction colorée utilisant un ester de l'indoxyle et d'un acide carbonique. Or, les esters d'indoxyle divulgués dans cet article présentent l'inconvénient d'être fortement instables du fait de taux élevés d'hydrolyse spontanée qu'ils présentent.

La présente invention a pour objet un agent de diagnostic permettant de déterminer la présence de leucocytes dans les urines, cet agent étant stable, permettant l'obtention d'un diagnostic rapide.

L'agent de diagnostic, objet de la présente invention comporte un tube à essai transparent renfermant au moins une bille, et, sous forme solide, au moins un ester de N-méthylindoxyle obtenu à partir d'acide caroxylique en $C_2$ à $C_5$.

L'ester de N-méthylindoxyle peut être fixé sur la surface externe de la bille mais, de préférence selon la présente invention, ledit ester est fixé sur la surface de la paroi intérieure du tube.

Il est bien entendu que dans le cadre de la présente invention, ledit ester peut être fixée simultanément sur la surface externe de la bille et sur la paroi interne du tube. L'ester tout particulièrement préféré selon la présente invention est l'acétate de N-méthylindoxyle.

Dans tous les cas, le tube à essai utile à l'agent de diagnostic selon la présente invention doit être formé d'un matériau transparent tel qu'un matériau plastique, notamment du polystyrène, ou tel que du verre.

De la même façon, la bille, utilisée dans le cadre de la présente invention, est de préférence transparente ou translucide. Elle peut être en matière plastique, en polystyrène notamment, mais est de préférence en verre.

Le diamètre des billes utilisées est, bien évidemment, inférieur au diamètre du tube à essai les incorporant. De préférence, selon la présente invention, le diamètre desdites billes est compris entre 1 et 15 mm et est en particulier inférieur à 10 mm.

L'agent de diagnostic selon la présente invention peut comporter, en outre, un agent tampon à base de phosphate. Il semble en effet qu'un tel agent permette d'accélérer le temps de réponse du test. On peut incorporer cet agent tampon sous une forme solide, éventuellement lyophilisée, soit au niveau de la ou des billes, soit au niveau de la paroi interne du tube à essai. On peut aussi utiliser cet agent tampon sous la forme d'une solution aqueuse que l'on introduit dans le tube juste avant la lecture du test.

La présente invention a encore pour objet un procédé de préparation dudit agent de diagnostic comportant les étapes consistant à imprégner la ou les billes et/ou la paroi intérieure du tube avec une solution dudit ester de N-méthylindoxyle dans un solvant approprié, puis à sécher la ou les billes et/ou la paroi intérieure du tube. Le séchage est destiné à fixer l'ester, à l'état solide, sur la ou les billes et/ou sur la paroi intérieure du tube.

De préférence, selon la présente invention, le solvant est formé d'alcool éthylique et il contient une proportion desdits esters comprise entre 8 mg/ml d'alcool éthylique et la teneur à saturation dans cet alcool ; la proportion tout particulièrement préférée étant inférieure à 45 mg/ml d'alcool éthylique. On peut utiliser de l'alcool éthylique à 95% v/v, mais, de préférence selon la présente invention, on utilise de l'éthanol absolu.

Le séchage de la ou des billes peut être réalisé par différents procédés connus de l'homme de l'art. Mais de préférence selon la présente invention, on utilise un courant froid d'un gaz, inerte vis-à-vis de l'ester fixé, dans lequel on place la ou les billes préalablement imprégnées par la solution alcoolique.

Le séchage de la paroi intérieure du tube pose, quant à lui, des problèmes très délicats que la présente invention a permis de résoudre. En effet, selon le procédé de la présente invention, après avoir versé une solution alcoolique de l'ester dans le tube à essai, on réalise le séchage du tube par évaporation de l'alcool sous vide puis on cristallise ledit ester en surfusion par addition, dans le tube de ladite ou desdites billes de la présente invention. L'ester se fixe ainsi sur la paroi intérieure du tube et/ou sur la surface externe de la ou des billes.

L'ester, tout particulièrement préféré pour la réalisation du procédé selon la présente invention est un acétate.

L'agent de diagnostic selon la présente invention est notamment utile pour déterminer la présence de leucocytes dans les urines ; il suffit pour cela, de verser dans ledit tube contenant la ou les billes, une faible quantité d'urine puis d'agiter légèrement le tube ; l'éventuelle coloration verte obtenue après repos est caractéristique de la présence de leucocytes.

En fait, le caractère transparent et translucide des billes est facultatif, mais avantageux dans la mesure où il favorise une lecture facile du test de détermination.

En outre, la présence de ces billes, lors de l'agitation du tube, permet une répartition homogène et rapide de la coloration verte dans le tube. Cette répartition homogène est notamment obtenue grâce à l'abrasion mécanique produite par les billes. Lesdites billes permettent en effet, lors de l'agitation, un détachement de l'ester des parois du tube et donc une mise en suspension dudit ester. Le produit coloré hydrosoluble obtenu après l'action des enzymes leucocytaires, est alors réparti rapidement et uniformément dans le tube.

Enfin, lors de la fabrication de l'agent de diagnostic selon l'invention, les billes favorisent l'obtention de l'ester sous forme solide.

En effet, lorsque l'on sèche dans un courant d'air froid les billes préalablement trempées dans une solution alcoolique d'acétate de N-méthylindoxyle, ce dernier se cristallise par adsorption sur la surface externe desdites billes.

De même, lorsque l'on additionne les billes à l'ester en surfusion dans les tubes à essai, préalablement séchés par évaporation de l'alcool sous vide, ledit ester, au contact desdites billes, se cristallise puis se fixe, par adsorption sur la surface de la paroi interne des tubes et/ou sur la surface externe des billes.

D'autres caractéristiques et avantages de l'invention apparaîtront à la lecture de la description suivante et des exemples donnés à titre illustratif et non limitatif.

## EXEMPLE 1

On prépare des billes de 6,4 mm de diamètre par trempe dans une solution formée de 750 mg d'acétate de N-méthylindoxyle/20 ml d'alcool éthylique à 95% v/v, le rapport du volume bille/solution = 1/10.

On laisse l'imbibition se poursuivre à l'abri de la lumière pendant 30 minutes puis, après séparation des billes, on les sèche dans un courant d'air froid.

On plonge une de ces billes dans un tube ou éprouvette dans lequel on verse un volume de 250 µl d'urine pour couvrir la bille, le diamètre du tube ou éprouvette employé étant d'environ 9 mm.

Après une demi-heure, on obtient une coloration verte, qui caractérise la présence des enzymes leucocytaires révélatrices de l'infection urinaire.

Un test témoin éventuel effectué sur bille identique traitée sur une urine ayant été chauffée pendant 2 minutes par un bain d'eau bouillante pour détruire toute activité enzymatique, montre qu'aucun changement de coloration ne se produit.

## EXEMPLE2

On prépare des billes de 8,0 mm de diamètre par trempe dans une solution formée de 450 mg d'acétate de N-méthylindoxyle/20 ml d'alcool éthylique à 95% v/v, le rapport du volume bille/solution = 1/17. On laisse l'imbibition se poursuivre à l'abri de la lumière pendant 30 minutes puis, après séparation des billes, on les sèche dans un courant d'air froid.

On plonge une de ces billes dans un tube ou éprouvette dans lequel on verse un volume de 350 µl d'urine pour couvrir la bille, le diamètre du tube ou éprouvette employé étant d'environ 11 mm.

Après une demi-heure, on obtient une coloration verte, caractéristique des enzymes leucocytaires dont la présence est recherchée dans les urines infectées.

Un test témoin éventuel effectué sur bille identique traitée sur une urine ayant été chauffée pendant 2 minutes par un bain d'eau bouillante pour détruire toute activité enzymatique, montre qu'aucun changement de coloration ne se produit.

## EXEMPLE 3

On opère dans les mêmes conditions que dans l'exemple 1 précité à la différence cependant, qu'en même temps que l'urine infectée, sont versés dans le tube ou éprouvette contenant la bille également 20 µl d'une solution 20 µM d'un tampon phosphate de pH = 7,5.

On constate que le temps pour l'obtention de la couleur obtenue dans l'exemple 1 est ramené à 20 minutes.

## EXEMPLE 4

On répartit une solution d'éthanol absolu concentrée à 1% v/v en acétate de N-méthylindoxyle, dans des tubes à essai en verre dont le diamètre est d'environ 10 mm, à raison de 40 µl par tube. L'alcool est évaporé sous vide. L'addition d'une bille de verre de 4 mm de diamètre permet la cristallisation de l'acétate de N-méthylindoxyle en surfusion.

Ainsi, 400 µg d'acétate de N-méthylindoxyle sont répartis de manière exacte dans chaque tube. On verse alors un volume d'urine d'environ 250 µl par tube. Une coloration verte apparaît environ 25 minutes après. Un test témoin est effectué dans les mêmes conditions que dans les exemples précédents. Aucun changement de coloration n'y apparaît.

## EXEMPLE 5

On opère dans les mêmes conditions que dans l'exemple 4, à l'exception de la bille de verre que l'on remplace par une bille en polystyrène dont le diamètre est d'environ 5 mm.

Une coloration verte apparaît en 25 minutes environ. La coloration obtenue est, dans certains cas, moins intense que celle obtenue à l'exemple 4 ; cette diminution de l'intensité peut s'expliquer par l'adsorption d'une quantité plus ou moins importante de l'ester sur la bille.

## EXEMPLE 6

On opère dans les mêmes conditions que dans l'exemple 5. La bille de polystyrène utilisée est préalablement imprégnée d'une solution 30 µM d'un tampon phosphate de pH 7.

On constate que le temps pour l'obtention de la couleur, obtenue dans l'exemple 5, est ramené à 16 minutes.

## EXEMPLE 7

On opère dans les mêmes conditions que dans les exemples 1 à 6 précités à la différence cependant, que l'acétate de N-méthylindoxyle est remplacé par le propylate de N-méthylindoxyle.

On constate que le temps pour l'obtention de la couleur est de l'ordre de ceux obtenus dans les exemples correspondants 1 à 6.

## Revendications

1. Agent de diagnostic, en particulier pour déceler la présence de leucocytes lors d'infections urogénitales, caractérisé en ce qu'il comporte un tube à essai transparent renfermant au moins une bille et, sous forme solide, au moins un ester de N-méthylindoxyle obtenu à partir d'acide carboxylique en $C_2$ à $C_5$.

2. Agent de diagnostic selon la revendication 1, caractérisé en ce que ledit ester est fixé sur la surface externe de la bille et/ou sur la surface de la paroi intérieure du tube.

3. Agent de diagnostic selon la revendication 1 ou 2, caractérisé en ce que la bille est transparente ou translucide.

4. Agent de diagnostic selon l'une des revendications 1 à 3, caractérisé en ce que la bille et/ou le tube est en verre.

5. Agent de diagnostic selon l'une des revendications 1 à 3, caractérisé en ce que la bille et/ou le tube est en matière plastique, en particulier en polystyrène.

6. Agent de diagnostic selon l'une des revendications 1 à 5, caractérisé en ce que ledit ester consiste en acétate de N-méthylindoxyle.

7. Agent de diagnostic selon l'une des revendications 1 à 6, caractérisé en ce qu'il comporte en outre un agent tampon à base de phosphate.

8. Procédé de préparation d'un agent de diagnostic selon l'une des revendications 1 à 7, caractérisé en ce qu'il comporte les étapes consistant à imprégner la ou les billes et/ou la paroi intérieure du tube avec une solution dudit ester de N-méthylindoxyle dans un solvant approprié, puis à sécher la ou les billes et/ou la paroi intérieure du tube, en vue d'y fixer l'ester sous forme solide.

9. Procédé de préparation de l'agent de diagnostic selon la revendication 8, caractérisé en ce que le solvant est formé d'alcool éthylique et en ce qu'il contient une proportion dudit ester comprise entre 8 mg/ml d'alcool éthylique et la teneur à saturation dans cet alcool, ladite proportion étant de préférence comprise entre 8 et 45 mg/ml d'alcool éthylique.

10. Procédé de préparation de l'agent de diagnostic selon la revendication 8 ou 9, caractérisé en ce qu'après avoir versé une solution alcoolique de l'ester de N-méthylindoxyle dans le tube à essai, on évapore l'alcool sous vide puis on cristallise ledit ester en surfusion par addition dans le tube d'au moins une bille.

11. Procédé de préparation de l'agent de diagnostic selon l'une des revendications 8 à 10, caractérisé en ce que ledit ester est un acétate.

Office européen
des brevets

**RAPPORT DE RECHERCHE EUROPEENNE**

Numéro de la demande

EP 87 40 0374

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | DE-A-2 129 661 (BOEHRINGER MANNHEIM) * Page 5, lignes 21-24; revendication 1 * | 1,5 | C 12 Q 1/34 |
| D,Y | CHEMICAL ABSTRACTS, vol. 96, no. 5, 1er février 1982, page 239, résumé no. 30471d, columbus, Ohio, US; E. VORMITTAG et al.: "Detection of leukocytes in urine by means of a test strip", & ASEAN J. CLIN. SCI. 1980, 1(3), 255-9 * Résumé * | 1,5 | |
| D,X | CHEMICAL ABSTRACTS, vol. 69, no. 1, 1er juillet 1968, page 22, résumé no. 255m, Columbus, Ohio, US; G.G. GUILBAULT et al.: "N-Methylindoxyl esters as substrates for cholinesterase", & ANAL. LETT. 1968, 1(6), 365-79 * Résumé * | 1,6 | **DOMAINES TECHNIQUES RECHERCHES (Int. Cl.4)** C 12 Q |
| A | FR-A-2 415 304 (ABBOTT LABORATORIES) * Page 2, lignes 12-17; page 6, revendications 1,2,6 * | 1,8 | |
| A | US-A-3 378 463 (G.G. GUIBAULT et al.) | | |

Le présent rapport de recherche a été établi pour toutes les revendications

| Lieu de la recherche | Date d'achèvement de la recherche | Examinateur |
|---|---|---|
| LA HAYE | 19-06-1987 | MEYLAERTS H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82